# EUROPEAN PATENT APPLICATION

(11) **EP 2 995 312 A1**
(43) Date of publication of application: **16.03.2016**
(21) Application number: 15175692.1
(22) Date of filing: 29.03.2004
(51) Int. Cl.: A61K 33/24, A61K 31/28, A61P 29/00

(54) **ANTI-INFLAMMATORY COMPOSITION BASED ON STRONTIUM COMPOUNDS**

(30) Priority: 27.03.2003 GB 0307137
(62) Divisional of application: 04724056.9
(71) Applicant: SantoSolve AS, 0319 Oslo (NO)
(72) Inventor: JELLUM, Egil, N-0319 Oslo (NO); FAGERLUND, Bjorn, Jari, N-0319 Oslo (NO); KJOLBERG, Clas, Magne, N-0319 Oslo (NO); KLAVENESS, Jo, N-0319 Oslo (NO); EGE, Thorfinn, N-0319 Oslo (NO)
(74) Representative: Price, Phillip

(57) **Abstract**

The invention provides a method of treatment of a human or non-human animal subject to combat inflammation arising from a condition associated with pain or a condition not associated with pain, said method comprising administering to said subject an effective amount of a physiologically tolerable strontium compound.

## Description

This invention relates to methods of anti-inflammatory treatment using strontium compounds, and to the use of strontium compounds for the manufacture of medicaments for use in such methods.

The use of strontium compounds to treat sub-dermal soft tissue pain is disclosed in our international patent application WO 03/028742 the contents of which are incorporated herein by reference.

We have now surprisingly found that strontium compounds may be used to achieve an anti-inflammatory effect both in conditions associated with pain and in conditions not associated with pain, and that in the former case the anti-inflammatory effect may beneficially occur at inflammation-affected sites which are distinct from the sites at which the pain is located, e.g. subdermal inflammation affected sites associated with psoriasis, herpetic infection (e.g. herpes simplex or herpes zoster), sun-burn, and acne vulgaris. Examples of conditions which result in inflammation but without necessarily involving an associated pain include polymyositis, dermamyositis, rheumatoid arthritis, osteoarthritis, sports injury, tension and over-use or misuse-induced muscle and tendon inflammation.

Further examples of inflammatory conditions treatable according to the invention include swelling and bruising associated with impact, psoriatic arthritis, and radiotherapy.

Such inflammation-associated conditions may be treated according to the invention by the administration of physiologically tolerable strontium compounds, e.g. by topical or oral administration or other gastrointestinal delivery routes, or more preferably by transdermal administration, for example injection, bolus injection into muscle, and insertion of extended release depot compositions (e.g. into muscle tissue). While the non-particulate compositions described below are especially suitable, it is also especially preferred to use compositions containing strontium in particulate form, e.g. particles of a strontium compound (optionally together with a matrix material such as for example a polymer), liposomes or other fragmented liquid crystalline forms containing the strontium compound in particulate, or more preferably dissolved form (e.g. in aqueous solution, membrane bound, or in lipid solution) and matrix particles (e.g. water swellable or erodible matrices such as polymer matrices) containing the strontium compound in dispersed form, e.g. microcrystalline or dissolved form.

Thus viewed from one aspect the invention provides the use of a physiologically tolerable strontium compound for the manufacture of a medicament for use as an anti-inflammatory, e.g. in the treatment of a condition associated with pain or of a condition not associated with pain.

Viewed from another aspect the invention provides a method of treatment of a human or non-human animal subject to combat inflammation arising from a condition associated with pain or a condition not associated with pain, said method comprising administering to said subject an effective amount of a physiologically tolerable strontium compound.

In a particularly preferred embodiment of these aspects of the invention the inflammation site is preferably sub-dermal and in soft tissue, e.g. in the torso or limbs, especially the muscles and tendons.

In a more particularly preferred embodiment of these aspects of the invention the inflammation is not associated with a sporting injury. In an especially preferred embodiment the inflammation is not associated with the mouth and administration of strontium is not into the mouth.

Dermal application of strontium chloride formulations containing 25% DMSO has now been shown to be effective for reducing inflammation in patients with a variety of diseases. However, in overweight patients and patients where the inflammation originates at a considerable distance from the application site, the strontium may be administered transdermally or surgically to a site below the dermal penetration barrier, e.g. in the form of an injection formulation, or in the form of a strontium releasing device.

Injection formulations of strontium can be nontoxic formulations for injection into any site below the dermal penetration barrier, and from which the strontium distributes by passive diffusion to reach the relevant pain receptors or neurons. Examples of the simplest forms of such formulations are strontium chloride in water, or in an isotonic solution. More complex formulations may contain agents like glycofurol and/or DMSO in order to increase diffusion from the site of its deposition. Such formulations may also contain excipients that cause it to function as a depot at the injection site, releasing the active pharmaceutical ingredient over an extended period of time. Alternatively, an injection formulation could also contain strontium in a form that causes it to accumulate in an organ like the liver, from which the strontium will gradually leak out and eventually reach its intended site of action. Particulate or lipophilic strontium products or high molecular weight carriers such as biological or synthetic soluble macromolecules are examples of formulations that will accumulate in the liver following intravenous injections. Examples of particulate strontium compounds that may be administered include strontium carbonate, strontium phosphate and strontium sulphate as well as liposomes or other fractured liquid crystalline phases containing a dissolved strontium compound, e.g. the chloride, in an internal aqueous phase. Examples of lipophilic strontium compounds include complexes of strontium with lipophilic complexing agents, e.g. those proposed for use as gadolinium complexing agents in the field of MR imaging, e.g. Schering AG (see current promotional material for Schering AG's MRI contrast media). Such lipophilic compounds may also be formulated in particulate products, e.g. micelles, liposomes, or fragmented cubic or hexagonal phases, e.g. in membrane-associated form or dissolved in a lipid phase.

In order to secure a sufficient high strontium concentration to function as an anti-inflammatory agent, simple strontium formulations may also be injected at or near its intended site of action. Examples of such administrations are injections into a sub-dermal organ, e.g. muscles or ligaments. Such formulations can also be placed into cavities directly in contact with the affected tissue like the bladder in patients with interstitial cystitis, or into the knee of patients with rheumatism in this site. More complex formulation could also contain strontium in a form that will case it to accumulate in the affected organ. Examples of the latter type of formulations are particulate or lipophilic strontium formulations that will accumulate in the liver following intravenous injections.

The strontium compound used according to the invention is preferably non-radioactive. By "non-radioactive" it is meant herein that the strontium compound is not so enriched in radioactive strontium isotopes as to qualify as a radioactive material for medical purposes. While a minute proportion of the strontium present in the strontium compound may of course be radioactive, the radioactive strontium isotope content of the strontium compound should generally be no more than 1000 times the natural abundance, preferably no more than 100 times, more preferably no more than 5 times. Most preferably the strontium compound contains radioactive strontium isotopes in no more than their natural abundances.

The strontium compound used according to the present invention may be any physiologically tolerable strontium compound capable on administration of acting as a source of strontium ions. Typically, the compound will be an inorganic or organic salt or a complex, e.g. with a chelating agent. Preferably the chelating agent of the strontium compound is present in excess by at least 2 % mol, more preferably by at least 50% mol, especially by at least 100% mol relative to the strontium. The excess chelating agent may have the function of binding calcium and thereby preventing transchelation and release of strontium. Besides small molecular chelating agents strontium can also be carried by natural or synthetic binding entities or substances or binding agents bound to carriers e.g. macromolecules such as proteins, polysaccharides, polyalkylene oxides, etc. Examples of preferred compounds include chloride, nitrate, sulphate, malate, citrate, lactate, oxalate, malate, fumarate, tartrate, malonate, acetate, gluconate, glutaconate, p-aminohippurate, succinate, phosphate, hydrogenphosphate, glycerophosphate, aminocaproate, mandelate, dibenzoyltartrate, stearate, ascorbate, benzoate, 3,4-dimethoxybenzoate, ranelate and methotrexate, and complexes with penicillamine, tyrosine, leucine, etc. Especially preferably the strontium compound, if in salt form, is in the form of the chloride, nitrate, acetate, citrate, lactate or hydrogenphosphate, particularly the chloride, acetate, citrate, lactate or hydrogenphosphate, more particularly the chloride. However the strontium compound may alternatively be present in the form of a chelate complex, e.g. with a polycarboxylic acid or polyphosphoric acid compound or a cyclic polyether. Examples of appropriate chelating agents are well known in the fields of nuclear medicine and magnetic resonance imaging (see for example the scientific and patent literature from Amersham, Nycomed, Schering, Salutar, Bracco, Sterling Winthrop, Mallinckrodt, etc). The use of linear or cyclic polychelants, such as EDTA, DTPA, EGTA, DTPA-BMA, DOTA, DO3A, 1,2-di(aminoethoxy)ethane-N,N,N',N'-tetraacetic acid, Kryptofix 5 and Kryptofix 222, especially EDTA, is particularly preferred.

It is especially preferred that the strontium compound be administered together with a further analgesic, e.g. aspirin, ibuprofen, or other NSAIDs or COX-2 inhibitors, or as a salt or complex of such an analgesic.

If desired the strontium compound may be administered as a salt or complex of a drug compound having an acid or amine group, preferably such a compound with a physiological effect beneficial to a complaint suffered by the patient, e.g. one effective at treating the underlying condition responsible for the pain. In the case of amino drugs, the resulting strontium compound might typically be a strontium chelate having the amino drug as a counterion. Examples of such drug compounds include nystatin, mesalazin, sulfasalazin, olsalazin, glutaminic acid, repaglinid, pantotenic acid, epoprostenol, iloprost, tirofiban, tranexamic acid, folic acid, furosemide, bumetanide, kanrenoic acid, capopril, enalapril, lisinopril, ramipril, fosinopril, trandolapril, valsartan, telmisartan, pravastatin, fluvastatin, atorvastatin, cerivastatin, sulfadiazin, tretinoin, adapalen, azelaic acid, dinoproston, levotyroxin, lityronin, doxycyclin, lymecyclin, oxytetracyclin, tetracyclin, ampicillin, amoxicillin, mecillinam, benzylpenicillin, phenoxymethylpenicillin, diclosacillin, clocsacillin, piperacillin, clavulanic acid, tazobactam, cefaleksin, cefalotin, cefoxitin, cefuroksim, ceftazidim, ceftriaxon, aztreonam, meropenem, imipenem, cilastatin, ciprafloksasin, nalidiksinic acid, fusidenic acid, phoscarnet, and zanamivir.

Various of the strontium compounds useful in the present invention include salts or complexes of strontium with cyclooxygenase inhibitors (other than salicylates (e.g. acetyl salicyclic acid) and oxicams (e.g. piroxicam and tenoxicam)), with amino acids, and with multidentate chelating agents (other than EDTA or EGTA) having the ability to form greater than 3, preferably greater than 4 metal coordination bonds.

Examples pf appropriate cyclooxygenase inhibitors (e.g. COX1 and/or COX2 inhibitors) include NSAIDs such as amfenac, bendazac, bufexamac, cinmetacin, diclofenac etodolac, felbinac, fenbufen, fenoprofen, fentiazac, flufenamic acid, flunixin, flunoxaprofen, flurbiprofen, furprofen, ibuprofen, indomethacin, ketoprofen, lonazolac, loxoprofen, mefenamic acid, mofezolac, naproxen, and niflumic acid. The strontium salts or complexes can readily be prepared by reacting strontium carbonate with the acid form of these compounds in solution.

Examples of amino acids that may be used to form strontium compounds for use according to the invention include all the natural alpha amino acids, e.g. tyrosine, leucine, lysine, etc. As with the COX 2 inhibitors, the compounds may be prepared in solution using strontium carbonate and the amino acid. However, other strontium salts can also be used, e.g. the chloride, acetate and hydroxide.

Examples of chelating agents which can be used to produce strontium compounds for use in the present invention include those with a diethylenetriamine or tetraazacyclododecane backbone carrying at least one oxyacid (e.g. carboxylic or phosphoric acid) metal binding group on the backbone nitrogens, e.g. DTPA, DTPA-bismethylamide, DOTA, DO3A, hydroxypropyl-DO3A, etc. These are well known from the diagnostic imaging contrast agent field and once again the strontium compounds can readily be prepared in solution from strontium carbonate.

In general, the strontium compound will be administered in a pharmaceutical composition comprising at least one physiologically tolerable carrier or excipient. The strontium compound may constitute up to 100% wt of the composition, preferably 0.005 to 50% wt, more preferably 0.05 to 20% wt, especially 0.1 to 10% wt, in particular 0.1 to 3% wt. Conventional pharmaceutical, carriers and excipients may be used, e.g. solvents (e.g. water, ethanol, etc), tableting agents, gelling agents, preservatives, emulsifiers, redox agents (e.g. antioxidants), blowing agents, thickeners, viscosity modifiers, pH modifiers, etc.

The strontium compositions for use in the method of the invention may take any convenient administration form depending on the proposed mode of administration (e.g. oral, rectal, nasal, sub-lingual, intramuscular, intravenous, vaginal, transdermal, topical or by inhalation). Thus the compositions may for example be in the form of solutions, dispersions, suspensions, gels, liquid crystalline systems and liquid crystal precursors, emulsions, syrups, tablets, coated tablets, capsules, creams, pastes, unguents, salves, suppositories, sprays, powders, etc. For intravenous and intramuscular administration, solutions are preferred. For transdermal or topical administration, solutions, creams, pastes, unguents, emulsions and gels are preferred. For oral administration, solutions, syrups, tablets, coated tablets and capsules are preferred.

For topical administration, it is especially preferred that the compositions contain a skin penetration enhancer and strontium compositions containing such penetration enhancers are novel and form a further aspect of the invention.

Thus viewed from a further aspect the invention provides an anti-inflammatory topical pharmaceutical composition comprising a physiologically tolerable strontium compound, a physiologically tolerable carrier (e.g. an aqueous solvent, gel, paste emulsion or cream) and a physiologically tolerable skin penetration enhancing agent.

Examples of suitable skin penetration enhancing agents include propylene glycol laurate, propylene glycol monolaurate, propylene glycol monocaprylate, isopropyl myristate, sodium lauryl sulphate, dodecyl pyridinium chloride, oleic acid, propylene glycol, diethylene glycol monoethyl ether, nicotinic acid esters, hydrogenated soya phospholipids, essential oils, alcohols (such as ethanol, isopropanol, n-octanol and decanol), terpenes, N-methyl-2-pyrrolidine, alpha-tocopherol, polyethylene glycol succinate (TPGS), Tween 80 and other surfactants, dimethyl-beta-cyclodextrin and dimethylsulphoxide, especially DMSO.

For administration into the gastrointestinal tract or vagina, it is especially preferred that the composition contain a bioadhesive to promote prolonged contact of the composition with the mucous membranes.

The bioadhesive compositions of the invention preferably contain the strontium compound in micronized form.

Bioadhesive (i.e. mucoadhesive) agents which may be used in natural or synthetic, polyanionic, polycationic or neutral, water-soluble or water-insoluble form, but are preferably large (e.g. having a molecular weight of 500 to 3000 kDa. e.g. 1000 to 2000 kDa), water-insoluble cross-linked (e.g. containing 0.05 to 2%, e.g. 0.75 to 1.5% cross-linker by weight of the total polymer, prior to any hydration), water-swellable polymers capable of forming hydrogen bonds. Preferably the bioadhesives have a mucoadhesive force greater than 100, especially preferably greater than 120, particularly greater than 150, as assessed according to the method of Smart et al. J. Pharm. Pharmacol. 36: p295-299 (1984), expressed as a percent relative to a standard in vitro.

Appropriate bioadhesives include, but are not limited to poly(carboxylic acid-containing) based polymers, such as poly(acrylic, maleic, itaconic, citraconic, hydroxyethyl methacrylic or methaorylic) acid which have strong hydrogen-bonding groups, or derivatives thereof such as salts and esters. Alternatively, cellulose derivatives may be used such as methyl cellulose, ethyl cellulose, methylethyl cellulose, hydroxymethyl cellulose, hydroxyethyl ethyl cellulose, carboxymethyl cellulose, hydroxypropylmethyl cellulose or cellulose esters or ethers or derivatives or salts thereof. Other naturally occurring or synthetic polymers may also be used such as gums, e.g. xanthan gum, guar gum, locust bean gum, tragacanth gum, karaya gum, ghatti gum, cholla gum, psillium seed gum and gum arabic; clays such as montmorillonite clays, e.g. Veegum, attapulgite clay; polysaccharides such as dextran, pectin, amylopectin, agar, mannan or polygalactonic acid or starches such as hydroxypropyl starch or carboxymethyl starch; lipophilic formulations containing polysaccharides, e.g. Orabase (Bristol Myers Squibb); carbohydrates, optionally polysubstituted with groups such as sulphate, phosphate, sulphonate or phosphonate, e.g. sucrose octasulphate; polypeptides such as casein, gluten, gelatin, fibrin glue; chitosan (lactate or glutamate) or carboxymethyl chitin; glycosaminoglycans such as hyaluronic acid; metal or water soluble salts of alginic acid such as sodium alginate or magnesium alginate; schleroglucan; adhesives containing bismuth oxide or aluminium oxide; atherocollagen; polyvinyl polymers such as polyvinyl alcohols, polyvinylmethyl ethers, polyvinylpyrrolidone, polycarboxylated vinyl polymers (such as polyacrylic acids as mentioned above); polysiloxanes, polyethers; polyethylene oxides and glycols; polyalkoxides and polyacrylamides and derivatives and salts thereof.

Bioadhesives may also be used which bind to the epithelial cell layer lying below the mucous layer. This allows more specific and longer lasting adhesion due to the slower relative turnover of epithelial cells compared to mucous turnover (days rather than hours). Thus for example, receptor-mediated interactions may be achieved using plant or bacterial lectins, i.e. (glyco) proteins of non-immune origin which bind to polysaccharides or glycoconjugates, which specifically bind to sugar moieties of the epithelial cell membrane. Also so-called "reverse" lectins of mammals in which receptors on the epithelial cell binds to sugars of the agent which is added, may be used. Other bioadhesives (e.g. adhesion or invasion factors (e.g. bacterial adhesins or invasins which bind to integrins) from bacteria or viruses may be used to allow selectively for particular tissues, phenotypes, disorders etc. by binding to only certain epithelial cells.

The above described polymeric bioadhesives may also be cross-linked and may be in the form of copolymers. Preferably poly(acrylic acid) polymers (or copolymers, e.g. with di- or poly-functional allyl ethers or acrylates to make the polymer insoluble), which have preferably been cross-linked, e.g. using a polyalkenyl polyether, may be employed which have a high molecular weight and are thixotropic. Appropriate bioadhesives having this form are available commercially (e.g. from Goodrich) as polycarbophil, e.g. Noveon AA-1, Carbomer (Carbopol), e.g. Carbopol EX165, EX214, 434, 910, 934, 934P, 940, 941, 951, 974P and 1342.

Some of the preferred bioadhesives thus include, polyacrylic hydrogels, chitosan, polyvinyl alcohol, hydroxypropyl cellulose, hydroxypropyl methyl cellulose, sodium alginate, scleroglucan, xanthan gum, pectin, Orabase and polygalactonic acid.

One particularly effective method of transdermal delivery of strontium ions is iontophoresis, e.g. using an iontophoretic assembly comprising a cathode in electrical contact with a drug reservoir, characterized in that said drug reservoir contains a physiologically tolerable strontium compound.

In the iontophoretic assemblies of the invention, the cathode is preferably a silver electrode and the strontium compound is preferably strontium chloride as the electrode reaction in this way produces insoluble silver chloride. The drug reservoir preferably comprises an aqueous gel containing the strontium compound in dissolved form. The assembly furthermore preferably also comprises a passive skin contact electrode and an electrical power source, e.g. a battery.

The compositions described above can also be used to treat deep tissue pain which has no associated inflammation, for instance cancers and neuropathic pains in areas where dermal administrations do not supply sufficient strontium to the affected areas, e.g. by administration by injection of one of the compositions described herein. It is known that the presence of subdermal fat or scar tissues or of hollow space between the dermal administration site and the affected region or of an extensive vascular network limits the penetration of strontium to the affected area. The present invention therefore provides an injection formulation for the treatment of soft tissue pain without associated inflammation. This method and use forms a further aspect of the invention.

The invention is illustrated further in the following non-limiting Examples.

### Example 1

### Composition

A strontium-containing composition was prepared as a 0.1% wt solution of strontium chloride hexahydrate in water.

### Example 2

### Composition

A strontium-containing composition was prepared as a solution in water of 0.1% wt strontium chloride hexahydrate, 0.1% wt of magnesium chloride hexahydrate and 0.1% wt calcium chloride dihydrate.

### Example 3

### Production of strontium a (II) complex of ethylenediamine tetraacetic acid (SrEDTA)

A suspension of strontium carbonate (1.0 g, 6.77 mmol) and ethylenediamine tetraacetic acid (1.98 g, 6.77 mmol) in water (25 ml) was stirred at 70°C for 30 minutes. The clear solution was evaporated to dryness and dried in vacuo at ambient temperature. The title compound was isolated as a white crystalline material. Yield 2.79 g (109% calculated as anhydrous product). Melting point above 250°C.

### Example 4

### Production of the strontium (II) complex of ethylene-bis (oxyethylenenitrilo) tetraacetic acid (SrEGTA)

A suspension of strontium carbonate (1.0 g, 6.77 mmol) and ethylenebis(oxyethylenenitrilo)tetraacetic acid (2.58 g, 6.77 mmol) in water (25 ml) was stirred at 70°C for 6.5 hours. The solution became almost clear. The solution was filtered at room temperature and the filtrate was evaporated to dryness and dried in vacuo at ambient temperature. The title compound was isolated as a white crystalline material. Yield 1.54 g (49%).

### Example 5

### Production of strontium (II) salicylate

A suspension of strontium carbonate (1.0 g, 6.77 mmol) and salicylic acid (1.87 g, 13.5 mmol) in water (25 ml) was stirred for 4 hours. The solution became pale yellow and almost clear. The solution was filtered at room temperature and the filtrate was evaporated to dryness and dried in vacuo at ambient temperature. The title compound was isolated as a pale red powder. Yield 2.1 g (86%). Melting point above 300°C.

### Example 6

### Production of the strontium (II) complex of diethylenetriaminepentaacetic acid (SrDTPA)

A suspension of strontium carbonate (1.0 g, 6.77 mmol) and diethylenetriaminepentaacetic acid (2.67 g, 6.77 mmol) in water (25 ml) was stirred at 80°C for 19 hours. The insoluble part was filtered off at room temperature and the filtrate was evaporated to dryness and dried in vacuo at ambient temperature. The title compound was isolated as a white/pale yellow crystalline material. Yield 1.6 g (49%). Melting point approx. 250°C.

The title compound was also prepared in 51% yield using a similar procedure with strontium acetate instead of strontium carbonate.

### Example 7

### Production of the strontium (II) complex of L-ascorbic acid

A suspension of strontium carbonate (1.0 g, 6.77 mmol) and L-ascorbic acid (2.39 g, 13.5 mmol) in water (25 ml) was stirred at 80°C for seven hours. The mixture became yellow. The mixture was filtered at room temperature, and the filtrate was evaporated to dryness and dried in vacuo at ambient temperature. The title compound was isolated as a yellow powder. Yield 2.50g (78%). Melting point approx. 250°C.

### Example 8

### Production of the strontium (II) complex of L-ascorbic acid 6-palmitate

A solution of strontium chloride hexahydrate (0.32 g, 1.2 mmol) in water (3 ml) was added to a stirred solution of L-ascorbic acid 6-palmitate (1.0 g, 2.4 mmol) in ethanol/water (100 ml, 50:50 (volume)) at room temperature. The mixture was stirred for 5 minutes and the title compound was isolated by filtration and dried by freeze-drying. Yield 0.514 g (47%). White powder.

### Example 9

### Production of strontium ibuprofen salt

Ibuprofen (2.59 g, 12.5 mmol) was dissolved in water (100 ml) containing sodium hydroxide (0.503 g, 12.5 mmol). A solution of strontium chloride hexahydrate (1.68 g, 6.3 mmol) in water (5 ml) was added. The mixture was stirred for 10 minutes at room temperature and the title compound was isolated by filtration and dried. Yield 1.30 g (44%). Melting point >300°C.

### Example 10

### Production of strontium diclofenac salt

Diclofenac (0.35 g, 1.18 mmol) was dissolved in water/ethanol (30 ml, 50:50 (volume)) containing sodium hydroxide (24 mg, 0.59 mmol). A solution of strontium chloride hexahydrate (0.16 g, 0.59 mmol) in water (3 ml) was added. The mixture was stirred for 20 minutes, and the title compound was isolated by filtration and dried. Yield 0.122 g (15%).

### Example 11

### Preparation of strontium stearate

Stearic acid (2.97 g, 10.4 mmol) was dissolved in water/ ethanol (100 ml, 50:50 (volume)) containing sodium hydroxide (0.417 g, 10.4 mmol). The mixture was heated to 70°C and a solution of strontium chloride hexahydrate (1.39 g, 5.2 mmol) in water (3 ml) was added. The title compound was isolated by centrifugation of the formed precipitate. Yield 1.6 g (46%).

### Example 12

### Production of SrEDTA dimeglumine salt

Strontium EDTA (1 g, 2.65 mmol) (from Example 3) and N-methyl-D-glucamine (1.03g, 529 mmol) were dissolved in water (10 ml) and stirred at 70°C for 30 minutes. The mixture was filtered and the filtrate was evaporated to dryness and dried in vacuo at ambient temperature. The title compound was isolated as white crystalline material. Yield 0.722 g (36%).

### Example 13

### Production of strontium benzoate

Strontium carbonate (1.0 g, 6.77 mmol) and benzoic acid (1.65 g, 13.5 mmol) in water (30 ml) were stirred for 4 hours at 70°C. The mixture was filtered and the filtrate was evaporated to dryness and dried in vacuo at ambient temperature. The title compound was isolated. Yield 1.8 g (81%).

### Example 14

### Production of strontium glutarate

Strontium carbonate (1.0 g, 6.77 mmol) and glutaric acid (0.89 g, 6.77 mmol) in water (30 ml) were stirred over night at 70°C. The mixture was filtered and the filtrate was evaporated to dryness and dried in vacuo at ambient temperature. The title compound was isolated. Yield 1.23 g (83%).

### Example 15

### Production of strontium alanine salt

Strontium hydroxide octahydrate (1.0 g, 3.79 mmol) and L-alanine (0.67 g, 7.52 mmol) in water (30 ml) were stirred for 4 hours at room temperature. The mixture was filtered and the filtrate was evaporated to dryness and dried in vacuo at ambient temperature. Yield 0.81 g (63%).

### Example 16

### Production of strontium hippurate

Strontium carbonate (0.5 g, 3.39 mmol) and hippuric acid (1.215 g, 6.77 mmol) in water (30 ml) were stirred at 70°C for 5 hours. The mixture was filtered and the filtrate was evaporated to dryness and dried in vacuo at ambient temperature. Yield 1.25 g (83%).

### Example 17

### Production of a strontium chelate with 1,2-di(2-amino-ethoxy)ethane-N,N,N',N'-tetraacetic acid

Strontium carbonate (1.0 g, 6.77 mmol) and 1,2-di(2-amino-ethoxy)ethane-N,N,N',N'-tetraacetic acid (2.58 g, 6.77 mmol) in water (30 ml) were stirred at 85°C for 48 hours. The mixture was evaporated and the title compound dried in vaccuo at ambient temperature. Yield 2.55 g (81%).

The compounds of Examples 3 to 17 may be formulated for administration in any convenient form (e.g. gels, creams, solutions, tablets, etc) using conventional pharmaceutical carriers and excipients.

### Example 18

### Skin penetration composition

A strontium-containing composition was prepared by dissolving 40 g strontium chloride hexahydrate in 1000 ml solvent. The composition of the solvent was:
50% (volume) distilled water
25% (volume) Tetraglycol^{®} (glucofurol)
25% (volume) DMSO

### Example 19

### Solution for injection

Strontium EDTA dimeglumine salt (20 mg) from Example 12 was dissolved in a 0.9% sterile aqueous solution (10 ml) and filled in a 10 ml vial (injection vial with rubber stopper). The solution was sterilised by autoclaving. The solution contained 0.2 mg strontium per ml.

### Example 20

### Hydrogel containing ibuprofen strontium ascorbate and skin penetration enhancer

Strontium ascorbate (900 mg) from Example 7 and sodium lauryl sulphate (450 mg) were mixed into Ibux gel 5% (produced by Weifa AS, Oslo, Norway) using a mortar and pestle. (Ibux gel contains 5% ibuprofen in a hydrogel comprising hydroxyethylcellulose, benzylalcohol, isopropanol, sodium hydroxyl and purified water). The resulting gel contained 1.2% wt strontium.

### Example 21

### Hydrogen comprising ibuprofen and strontium chloride

Strontium chloride hexahydrate (0.8 g) was mixed into Ibux gel 5% (19.2 g) using a mortar and pestle.

### Example 22

### Mucoadhesive hydrogel comprising ibuprofen and strontium chloride

Polyacrylic acid 5100 sodium salt (Fluka 81132) (0.21 g) was mixed into a hydrogel comprising ibuprofen and strontium (see Example 21) (7.0 g) using a mortar and pestle.

### Example 23

### Mucoadhesive hydrogel comprising ibuprofen and strontium chloride

Strontium chloride hexahydrate (1.5 g) and chitosan malate (203-490-14SM from FMC Biopolymers, Drammen, Norway) (0.75 g) were mixed into Ibux gel 5% (12.75 g). The resulting mucoadhesive gel contained 3.3% wt strontium and 5% wt ibuprofen.

### Example 24

### Cream containing strontium chloride

Strontium chloride hexahydrate (1.2 g) was mixed into Unguentum Merck (13.8 g) using a mortar and pestle. The cream contained 2.6% wt strontium in the form of strontium chloride.

### Example 25

### Cream containing strontium chloride and a skin penetration enhancer

Strontium chloride hexahydrate (1.2 g) and sodium lauryl sulphate (0.3 g) were mixed into Unguentum Merck (13.5 g) using a mortar and pestle. The cream contained 2.6% wt strontium in the form of strontium chloride.

### Example 26

### Cream containing lidocaine and strontium diclofenac salt

Strontium diclofenac (40 mg) from Example 10 was mixed into Xylocain^{®} 5% cream (Astra Zeneca AS, Oslo, Norway) using a mortar and pestle. (100 g Xylocain^{®} 5% cream contains 5 g lidocaine in coconut oil 13.8 g, polyoxyethylene ester 4.5 g, carboxypolymethylene 1 g, sodium hydroxide 6.5 g and purified water 69 g). The cream contained 5% wt lidocaine and 40 mg/g strontium diclofenac.

### Example 27

### Ointment containing hydrocortisone and strontium stearate

Strontium stearate (60 mg) from Example 11 was mixed into Hydrokortison 1% ointment (Galderma Nordic AB) using a mortar and pestle (Hydrokortison 1% ointment contains 1% hydrocortisone, propyleneglycol, liquid paraffin, cetylalcohol and Vaseline^{®}). The resulting ointment contained 1% wt hydrocortisone and 3% wt strontium stearate.

### Example 28

### Mucoadhesive formulation containing strontium ibuprofen

Strontium ibuprofen (0.5 g) from Example 9 was mixed into Orabase^{®} paste (Squibb AB, Lidingö, Sweden) (14.5 g) using a mortar and pestle. Orabase^{®} contains gelatin, pectin, sodium carboxymethhylcellulose, polyethylene and liquid paraffin. The resulting formulation contained 3.3% wt strontium ibuprofen and is useful for treatment of pain in the mouth or other mucosal body surfaces.

### Example 29

### Preparation of nanoparticles comprising strontium EDTA

Poly(D,L-lactide-co-glycolide) (50:50) (M_{w} 20000) (100 mg) is dissolved in dichloromethane (10 mg). An aqueous solution of poly(vinyl alcohol) (PVA) (M_{w} 15000) (2.5%) (3 ml) containing strontium EDTA (10 mg) (from Example 4) is added and the mixture is homogenised for 10 minutes. An aqueous solution of PVA (1.5%) (25 ml) is added and the mixture is evaporated down. The resulting nanoparticles are washed with water and freeze dried.

### Example 30

### Preparation of suspension of nanoparticles comprising strontium EDTA for infection

Nanoparticles comprising strontium EDTA (100 mg) from Example 29 are dispersed in an isotonic aqueous solution of glucose (5 ml). The suspension contains 2 mg strontium EDTA per ml.

### Example 31

### Lipid emulsion of strontium stearate for injection

Strontium stearate (5 g) (from Example 11) is added to a lipid emulsion (Intralipid^{®} 300 mg lipid per ml from Pharmacia and Upjohn) (500 ml). The mixture is homogenized for 1 hour and filled into 10 ml vials. Each vial contains 100 mg strontium stearate.

### Example 32

### Lipid formulation of strontium ibuprofen for intramuscular injection (sustained release)

Strontium ibuprofen salt (50 mg) (from Example 9) is added to saturated triglyceride (1 ml). The mixture is homogenized for 2 minutes.

### Example 33

### Preparation of suspension of liposomes containing strontium chloride

Soy bean lecithin (0.8 g) is dissolved in ethanol (8 ml). The mixture is rotary evaporated into a thin film at 55°C. The lipid film is rehydrated with an aqueous solution of strontium chloride (50 mg in 6 ml) by shaking at 60°C for 20 minutes followed by sonication and extrusion through 100 nm polycarbonate filter. The extrusion is repeated 10 times. The mixture is filled into a 10 ml vial.

### Example 34

A 14 year old boy, suffering from severe acne vulgaris for more than a year, had been using cleansing creams, speciality soaps and Cliniderm cream without significant effects. Following topical treatment with the composition of Example 18 twice daily, significant improvement was observed on day 3 after commencement of the treatment, with further improvement being observed following further treatment.

### Example 35

A 50 year old female with a 12 year long history of severe psoriasis of hands, arms and feet had observed some effects following use of Daivonex and cortisone, but these effects did not last following long term treatment. Following daily treatment with the composition of Example 18, there was a rapid reduction of the pruritus in the effected areas, and within 2 days also a significant improvement in the appearance of the lesions. The improvement further increased following continued use of the composition, and the lesions could be kept at an insignificant level by continuous use of the product.

### Example 36

A 45 year old female with a 10 year history of severe psoriatic arthritis had been using intermittent cytostatic treatment to inhibit progression of the disease. Significant deformation of fingers was observed as a result of the disease. Following treatment with the composition of Example 18, a strong effect on the inflammation was observed, and could be maintained with daily administration of the composition.

### Example 37

Following radiation therapy for a head and neck cancer, the patient experienced a strong inflammatory and painful reaction in part of the radiation field. Following treatment with the composition of Example 18, a rapid analgesic effect was experienced, and this was followed by a delayed and strong anti-inflammatory effect. The effect was maintained by daily administration with the composition.

### Example 38

### Pharmaceutical formulation comprising strontium carbonate in lipid emulsion for injection (sustained release).

Strontium carbonate (200 mg) was suspended in a lipid emulsion (Intralipid Fresenius Kabi,200 mg/ml, 10 ml) by extensive sonication in a sonication bath for 1 hour. The homogenous suspension was filled into an injection vial. The suspension contained 11.8 mg strontium per ml in the form of strontium carbonate.

### Example 39

### Pharmaceutical formulation comprising of a suspension of strontium carbonate for injection (sustained release).

Strontium acetate (500 mg) was dissolved in an aqueous solution of sodium chloride (0.9%, 5 ml). A solution of sodium carbonate (250 mg) in an aqueous solution of sodium chloride (0.9%, 5 ml) was added dropwise to the stirred solution of strontium acetate. The mixture was stirred for 15 minutes and filled into a injection vial (10 ml). The vial contained a suspension of small particles of strontium carbonate. Each ml contained 21 mg strontium in the form of small particulates. The mixture should be shaken before injection. Other suspensions of strontium carbonate with different particle size and crystalline properties can be made by using other precipitation conditions. Addition of macromolecules and/or surface active compounds will have an effect on the crystalline properties of the precipitated strontium carbonate.

### Example 40

### Pharmaceutical formulation comprising strontium acetate and rofecoxib for injection.

Rofecoxib (70 mg, extracted from commercial Celebra tablets(Pharmacia, Pfizer)) and strontium acetate (150 mg) were dissolved in Intralipid (Fresenius Kabi 200 mg/ml, 10 ml) by extensive sonication in a sonication bath for 1 hour. The homogenous suspension was filled into an injection vial (10 ml). Each ml contained 7 mg rofecoxib and 6.4 mg strontium in the form of strontium acetate.

### Intermediate

### Precipitation and isolation of strontium carbonate particles.

Strontium carbonate particles were prepared by precipitation from strontium acetate and sodium carbonate in water as described in Example 39. The precipitated strontium carbonate was isolated by centrifugation, resuspended in water and isolated by centrifugation.

### Example 41

### Injection formulation of strontium for immediate and sustained release.

Strontium carbonate (200 mg, from the Intermediate in Example 40) and strontium acetate (50 mg) were dissolved/suspended in 0.9% sodium chloride (10 ml). The formulation contained both soluble strontium acetate for immediate release and strontium carbonate for sustained release of strontium.

### Example 42

### Injection formulation of strontium stearate in lipid emulsion.

Strontium stearate (6 mg, Example 11) was dissolved in Intralipid (Fresenius Kabi,200 mg/ml, 1 ml) by extensive sonication in a sonication bath for 20 minutes. The homogenous suspension was filled on a vial.

### Example 43

### Solution of strontium ibuprofen salt for injection.

Strontium ibuprofen salt (50 mg, Example 9) was dissolved in 0.9% sodium chloride (10 ml) by heating to 80 °C. The solution was filled into an injection vial.

### Example 44

### Solution of strontium chelate for injection.

Strontium EDTA dimeglumine salt (300 mg, Example 12) and EDTA calcium disodium salt hydrate (50 mg) were dissolved in aqueous glucose solution (B.Braun,Germany, 50 mg/ml, 10 ml).

### Example 45

Injection formulation of strontium compounds in lipid emulsion.

Strontium acetate (50 mg), strontium carbonate (200 mg, from the intermediate in Example 40) and strontium diclofenac (50 mg, Example 10) were suspended/dissolved in Intralipid (Fresenius Kabi,200 mg/ml, 10 ml) by extensive sonication in a sonication bath. The formulation contained a mixture of 3 different strontium compounds with different release profiles.

### Example 46

### Solution for injection comprising strontium ascorbate.

Strontium ascorbate (100 mg, Example 7) was dissolved in a solution of glucose (B.Braun,Germany,10 ml). The solution was pale yellow.

### Example 47

### Strontium alginate

Alginate (Protanal LF 120L, 300 mg) was dissolved in water (50 ml) by heating to 80°C. The mixture became very viscous. A solution of strontium acetate (200 mg) was added dropwise and a gel precipitated out. The solution was cooled and the gel removed from the solution.

Strontium alginate complexes can be prepared in different ways to obtain materials with different properties. A strontium alginate gel like material might be useful for i.v. administration for sustained release of strontium.

Embodiments of the invention are also described in the claims of the International application as filed:
1. A method of treatment of a human or non-human animal subject to combat inflammation arising from a condition associated with pain or a condition not associated with pain, said method comprising administering to said subject an effective amount of a physiologically tolerable strontium compound.
2. A method as claimed in claim 1 wherein said strontium compound is administered to the surface of the skin.
3. A method as claimed in either of claims 1 and 2 wherein said inflammation is not associated with pain.
4. A method as claimed in any one of claims 1 to 3 wherein said inflammation is not associated with a sporting injury.
5. A method as claimed in any one of claims 1 to 4 wherein said administration is not oral.
6. The use of a physiologically tolerable strontium compound for the manufacture of a medicament for use as an anti-inflammatory.
7. Use as claimed in claim 6 for the manufacture of a medicament for use in the treatment of a condition not associated with pain.
8. Use as claimed in either of claims 6 and 7 wherein said medicament further contains a skin penetration enhancing agent.
9. Use as claimed in claim 8 wherein said agent is dimethylsulphoxide.
10. Use as claimed in any one of claims 6 to 8 of a said strontium compound selected from strontium chloride, strontium acetate and strontium nitrate.
11. Use as claimed in any one of claims 6 to 10 wherein said strontium compound is a strontium complex, e.g. with a chelating agent.
12. Use as claimed in claim 11 wherein said chelating agent is present in at least 2% mol excess relative to strontium.
13. Use as claimed in claim 12 wherein said chelating agent is present in an excess of at least 50% mol relative to strontium.
14. Use as claimed in any one of claims 6 to 13 wherein said inflammation is associated with acne vulgaris.
15. Use as claimed in any one of claims 6 to 13 wherein said inflammation is associated with psoriasis.
16. Use as claimed in any one of claims 6 to 13 wherein said inflammation is associated with radiation therapy.
17. Use as claimed in any one of claims 6 to 13 wherein said inflammation is associated with arthritis.

## Claims

1. A method of treatment of a human or non-human animal subject to combat inflammation arising from a condition associated with pain or a condition not associated with pain, said method comprising administering to said subject an effective amount of a physiologically tolerable strontium compound.

2. A method as claimed in claim 1 wherein said strontium compound is administered to the surface of the skin.

3. A method as claimed in either of claims 1 and 2 wherein said inflammation is not associated with pain.

4. A method as claimed in any one of claims 1 to 3 wherein said inflammation is not associated with a sporting injury.

5. A method as claimed in any one of claims 1 to 4 wherein said administration is not oral.

6. The use of a physiologically tolerable strontium compound for the manufacture of a medicament for use as an anti-inflammatory.

7. Use as claimed in claim 6 for the manufacture of a medicament for use in the treatment of a condition not associated with pain.

8. Use as claimed in either of claims 6 and 7 wherein said medicament further contains a skin penetration enhancing agent.

9. Use as claimed in claim 8 wherein said agent is dimethylsulphoxide.

10. Use as claimed in any one of claims 6 to 8 of a said strontium compound selected from strontium chloride, strontium acetate and strontium nitrate.

11. Use as claimed in any one of claims 6 to 10 wherein said strontium compound is a strontium complex, e.g. with a chelating agent.

12. Use as claimed in claim 11 wherein said chelating agent is present in at least 2% mol excess relative to strontium.

13. Use as claimed in claim 12 wherein said chelating agent is present in an excess of at least 50% mol relative to strontium.

14. Use as claimed in any one of claims 6 to 13 wherein said inflammation is associated with acne vulgaris, psoriasis, radiation therapy or arthritis.
